# EUROPEAN PATENT SPECIFICATION

(11) **EP 3 054 953 B1**
(45) Date of publication and mention of the grant of the patent: **01.07.2020**
(21) Application number: 14852240.2
(22) Date of filing: 08.10.2014
(51) Int. Cl.: A61K 31/505, A61K 31/519, A61K 31/52, A61P 35/00, A61K 31/5377

(54) **HDAC INHIBITORS IN COMBINATION WITH PI3K INHIBITORS, FOR TREATING NON-HODGKIN'S LYMPHOMA**
HDAC-HEMMER IN KOMBINATION MIT PI3K-HEMMERN, ZUR BEHANDLUNG VON NICHT-HODGKIN-LYMPHOMEN
INHIBITEURS DE HDAC EN COMBINAISON AVEC DES INHIBITEURS DE PI3K, POUR LE TRAITEMENT DU LYMPHOME NON HODGKINIEN

(30) Priority: 10.10.2013 US 201361889207 P; 03.12.2013 US 201361911097 P
(43) Date of publication of application: 17.08.2016
(73) Proprietor: Acetylon Pharmaceuticals, Inc., Boston, MA 02210 (US)
(72) Inventor: QUAYLE, Steven Norman, Brookline, MA 02445 (US); JONES, Simon S., Harvard, MA 01451 (US)
(74) Representative: Boult Wade Tennant LLP
(86) International application number: PCT/US2014/059640
(87) International publication number: WO 2015/054355

(56) References cited:
- WO-A1-2015/054099
- WO-A2-2011/091213
- WO-A2-2011/091213
- WO-A2-2012/068109
- JONATHAN H SCHATZ: "Targeting the PI3K/AKT/mTOR Pathway in Non-Hodgkin's Lymphoma: Results, Biology, and Development Strategies", CURRENT ONCOLOGY REPORTS, CURRENT SCIENCE INC, NEW YORK, vol. 13, no. 5, 14 July 2011 (2011-07-14), pages 398-406, XP019946841, ISSN: 1534-6269, DOI: 10.1007/S11912-011-0187-7
- DE VOS SVEN ET AL: "A Phase 1 Study of the Selective Phosphatidylinositol 3-Kinase-Delta (PI3K delta) Inhibitor, Cal-101 (GS-1101), in Combination with Rituximab and/or Bendamustine in Patients with Previously Treated, Indolent Non-Hodgkin Lymphoma (iNHL)", BLOOD, THE AMERICAN SOCIETY OF HEMATOLOGY, & 53RD ANNUAL MEETING AND EXPOSITION OF THE AMERICAN-SOCIETY-OF-HEMATOLOGY (ASH); SAN DIEGO, CA, USA; DECEMBER 10 -13, 2011, vol. 118, no. 21, 13 December 2011 (2011-12-13), page 1160, XP008152289, ISSN: 0006-4971
- BODO, JURAJ ET AL.: 'The phosphatidylinositol 3-kinases (PI3K) inhibitor GS-1101 synergistically potentiates histone deacetylase inhibitor-induced proliferation inhibition and apoptosis through the inactivation of PI3K and extracellular signal- regulated kinase pathways.' BRITISH JOURNAL OF HAEMATOLOGY vol. 163, no. 1, October 2013, pages 72 - 80, XP055333061
- RAHMANI, MOHAMED ET AL.: 'Inhibition of PI-3 kinase sensitizes human leukemic cells to histone deacetylase inhibitor-mediated apoptosis through p44/42 MAP kinase inactivation and abrogation of p21CIP1/WAF1 induction rather than AKT inhibition.' ONCOGENE vol. 22, 2003, pages 6231 - 6242, XP055269982
- DENLINGER, CHADRICK E. ET AL.: 'Inhibition of phosphatidylinositol 3-kinase/Akt and histone deacetylase activity induces apoptosis in non-small cell lung cancer in vitro and in vivo.' THE JOURNAL OF THORACIC AND CARDIOVASCULAR SURGERY vol. 130, no. 5, November 2005, pages 1422 - 1429, XP005127283

## Description

### CROSS-REFERENCE TO RELATED APPLICATIONS

This application claims priority to U.S. Provisional Application Serial No. 61/889,207, filed October 10, 2013, and U.S. Provisional Application Serial No. 61/911,097, filed December 3,2013.

### BACKGROUND

WO 2011/091213 relates to reverse amide compounds as protein deacetylase inhibitors and methods of use thereof.

Histone deacetylase (HDAC) enzymes represent attractive therapeutic targets in non-hodgkin's lymphoma (NHL), but unfortunately non-selective HDAC inhibitors have led to dose-limiting toxicities in patients.

Phosphatidylinositide 3-kinases (PI 3-kinases, PI3Ks, PI(3)Ks, PI-3Ks, phosphatidylinositol-3-kinases, or phosphoinositide 3-kinases) are a family of enzymes involved in cellular functions, such as cell growth, proliferation, differentiation, motility, survival and intracellular trafficking, which in turn are involved in cancer. PI3Ks are a family of related intracellular signal transducer enzymes capable of phosphorylating the 3 position hydroxyl group of the inositol ring of phosphatidylinositol.

Due to the dose-limiting toxicities of the non-selective HDAC inhibitors, there is an ongoing need in the art for more efficacious and less toxic compositions and methods for the treatment of non-hodgkin's lymphoma. In order to meet these needs, provided herein are HDAC inhibitors, pharmaceutical combinations comprising an HDAC inhibitor and a phosphatidylinositide 3-kinase (PI3K) inhibitor, and methods for the treatment of non-hodgkin's lymphoma. The compounds, combinations, and methods of the invention are well tolerated and do not exhibit the dose-limiting toxicities of prior therapies.

### SUMMARY OF THE INVENTION

In the first aspect of the invention there is provided a pharmaceutical combination for use in treating non-hodgkin's lymphoma comprising a therapeutically effective amount of a histone deacetylase 6 (HDAC6) specific inhibitor, wherein the HDAC6 specific inhibitor is: 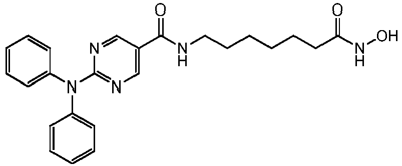 or a pharmaceutically acceptable salt thereof;
and a phosphatidylinositide 3-kinase (PI3K) inhibitor, wherein the PI3K inhibitor is GDC-0941 or GS-1101, or a pharmaceutically acceptable salt thereof.

In the second aspect of the invention there is provided a pharmaceutical combination for use in treating non-hodgkin's lymphoma comprising a therapeutically effective amount of a histone deacetylase 6 (HDAC6) specific inhibitor, wherein the HDAC6 specific inhibitor is: 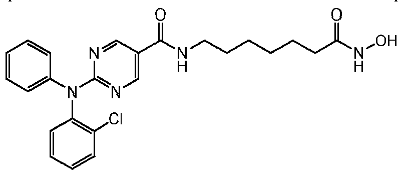 or a pharmaceutically acceptable salt thereof;
and a phosphatidylinositide 3-kinase (PI3K) inhibitor, wherein the PI3K inhibitor is GDC-0941 or GS-1101, or a pharmaceutically acceptable salt thereof.

Provided herein are pharmaceutical combinations for the treatment of non-hodgkin's lymphoma in a subject in need thereof. The invention is defined by the claims. Any subject matter included herein and falling outside the scope of the claims is provided for information purposes only.

In some embodiments, the combinations decrease cell viability, cell proliferation, and synergistically increases apoptosis of cells.

In specific embodiments, the HDAC6 specific inhibitor is: 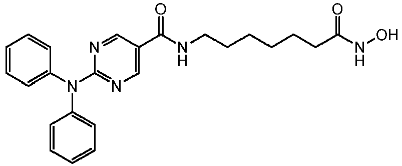 or a pharmaceutically acceptable salt thereof.

In yet other embodiments, the HDAC6 specific inhibitor is is: 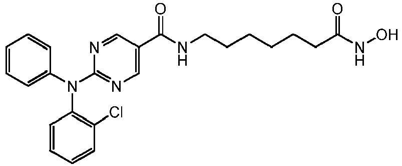 or a pharmaceutically acceptable salt thereof.

The phosphatidylinositide 3-kinase (PI3K) inhibitor is GDC-0941 or GS-1101, or pharmaceutically acceptable salts thereof.

In some embodiments, the HDAC inhibitor is administered with a pharmaceutically acceptable carrier. In other embodiments, the combination of the HDAC inhibitor and the phosphatidylinositide 3-kinase (PI3K) inhibitor is administered with a pharmaceutically acceptable carrier.

In some embodiments, the HDAC inhibitor and the phosphatidylinositide 3-kinase (PI3K) inhibitor are administered in separate dosage forms. In other embodiments, the HDAC inhibitor and the phosphatidylinositide 3-kinase (PI3K) inhibitor are administered in a single dosage form.

In some embodiments, the HDAC inhibitor and the phosphatidylinositide 3-kinase (PI3K) inhibitor are administered at different times. In other embodiments, the HDAC inhibitor and the phosphatidylinositide 3-kinase (PI3K) inhibitor are administered at substantially the same time.

In some embodiments, the combination of the HDAC inhibitor and the phosphatidylinositide 3-kinase (PI3K) inhibitor achieves a synergistic effect in the treatment of the subject in need thereof. In some embodiments, the combination of the HDAC inhibitor and the phosphatidylinositide 3-kinase (PI3K) inhibitor achieves a synergistic effect in the treatment of the subject in need thereof, wherein the combination is administered at dosages that would not be effective when one or both of the compounds are administered alone, but which amounts are effective in combination.

There is provided a method for decreasing cell viability of cancer cells by administering a histone deacetylase (HDAC) inhibitor, or a combination comprising a histone deacetylase (HDAC) inhibitor and a phosphatidylinositide 3-kinase (PI3K) inhibitor.

There is provided a method for synergistically increasing apoptosis of cancer cells by administering a combination comprising a histone deacetylase (HDAC) inhibitor and a phosphatidylinositide 3-kinase (PI3K) inhibitor.

There is provided a method for decreasing cell proliferation of cancer cells by administering a combination comprising a histone deacetylase (HDAC) inhibitor and a phosphatidylinositide 3-kinase (PI3K) inhibitor.

There is provided a method for reducing tumor growth by administering a combination comprising a histone deacetylase (HDAC) inhibitor and a phosphatidylinositide 3-kinase (PI3K) inhibitor.

There is provided a method for suppressing Myc expression by administering a combination comprising a histone deacetylase (HDAC) inhibitor and a phosphatidylinositide 3-kinase (PI3K) inhibitor.

Other objects, features, and advantages will become apparent from the following detailed description. The detailed description and specific examples are given for illustration only because various changes and modifications within the scope of the invention will become apparent to those skilled in the art from this detailed description. Further, the examples demonstrate the principle of the invention.

### BRIEF DESCRIPTION OF THE FIGURES

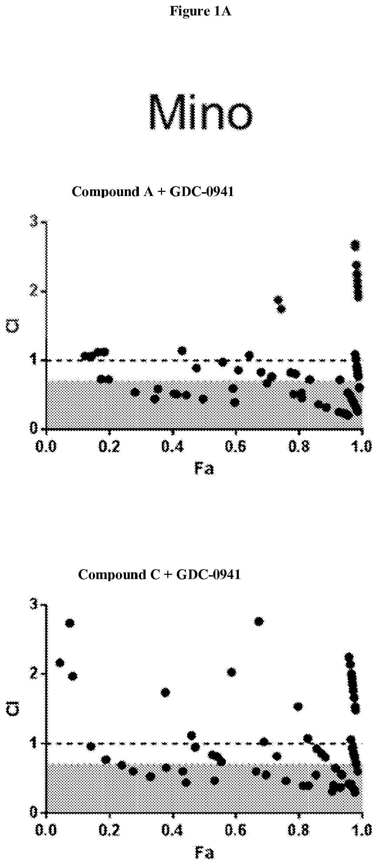

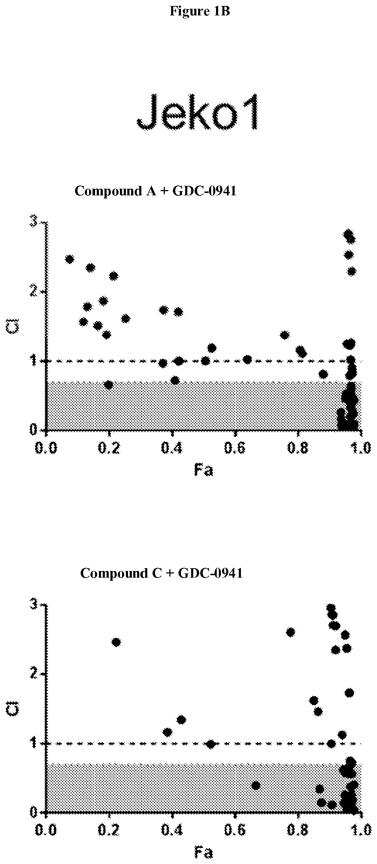

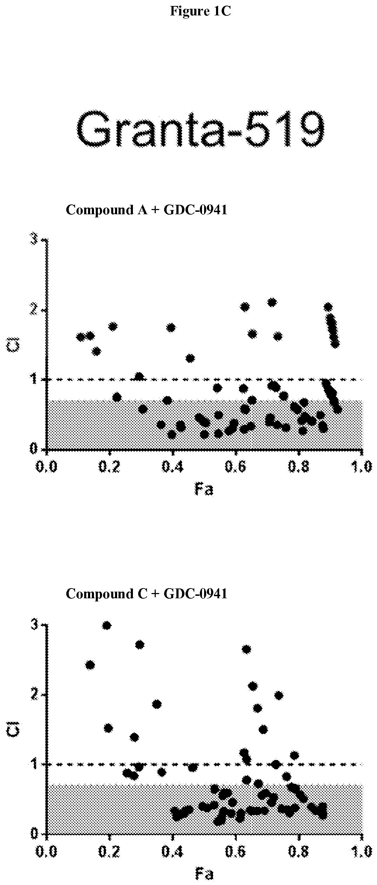

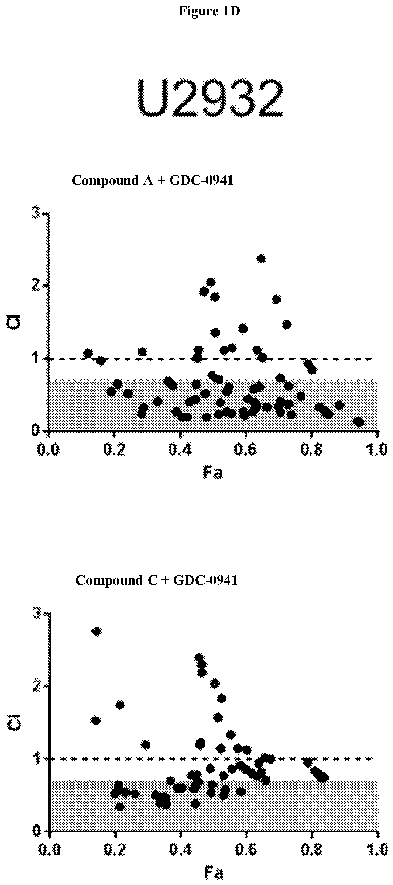

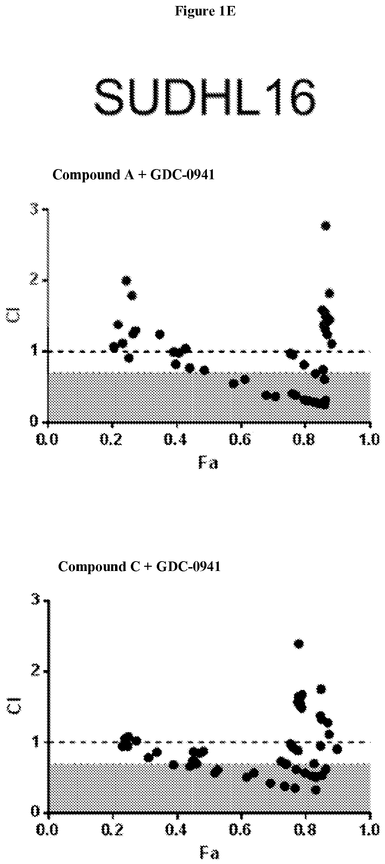

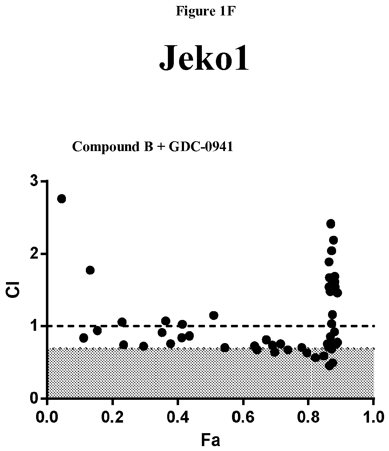

**Figures 1A-F** show F_{A}/CI Synergy Plots after treatment of human lymphoma cell lines with GDC-0941 (pictilisib) and either **Compound A, Compound B,** or **Compound C.** For Mantle Cell Lymphoma (MCL) Mino, Jeko1, and Granta-519 cells were utilized, while U2932 and SUDHL16 cells represented the activated B cell (ABC) and germinal center (GC) subtypes of diffuse large B cell lymphoma (DLBCL). Data points with Combination Index (CI) values <1 indicate treatment combinations resulting in synergistic decreases in cellular viability.

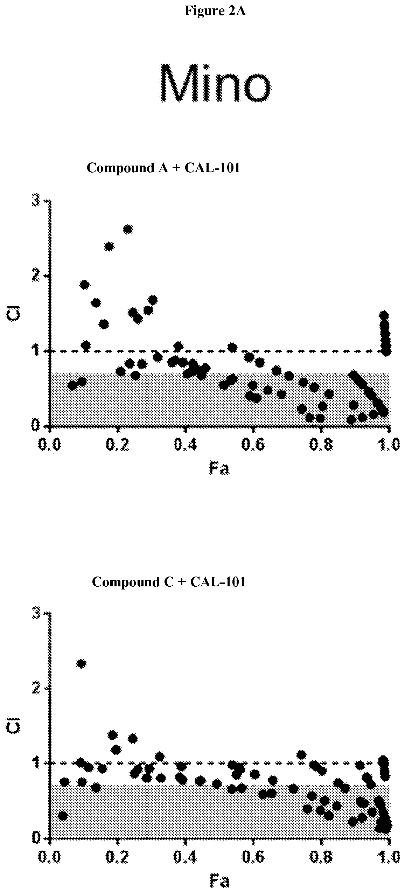

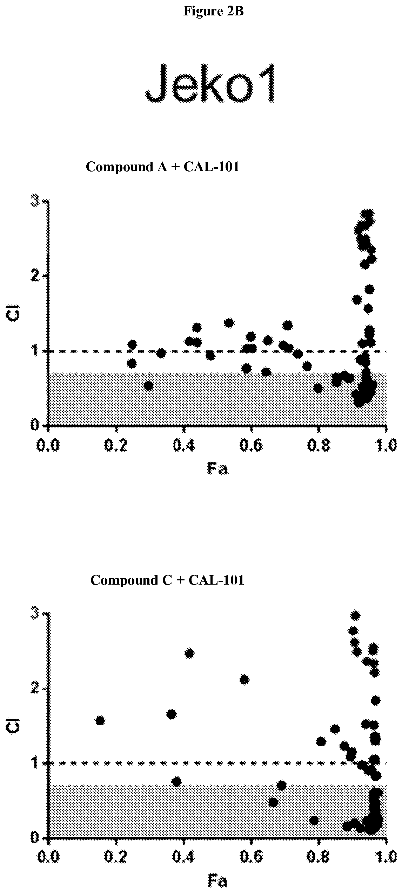

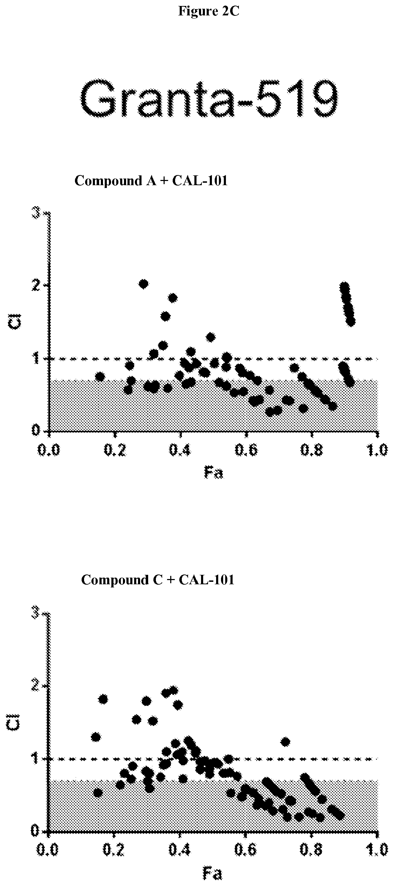

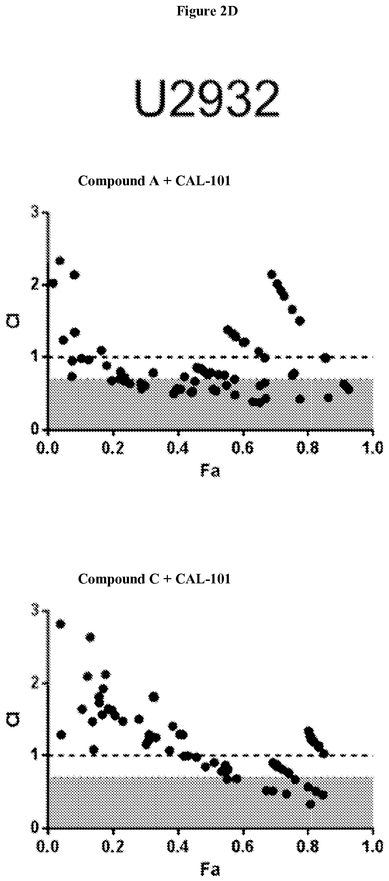

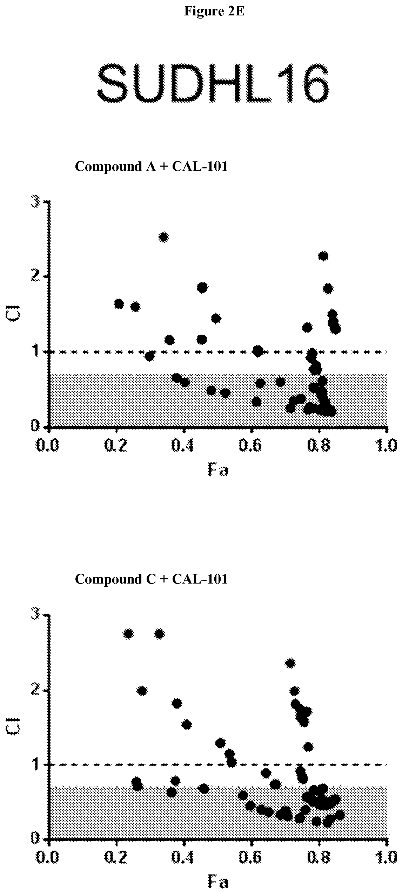

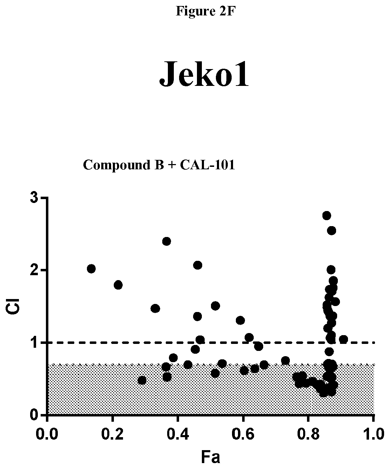

**Figures 2A-F** show F_{A}/CI Synergy Plots after treatment of human lymphoma cell lines with CAL-101 (GS-1101, idelalisib, Zydelig) and either **Compound A, Compound B,** or **Compound C.** For Mantle Cell Lymphoma (MCL) Mino, Jeko1, and Granta-519 cells were utilized, while U2932 and SUDHL16 cells represented the activated B cell (ABC) and germinal center (GC) subtypes of diffuse large B cell lymphoma (DLBCL). Data points with Combination Index (CI) values <1 indicate treatment combinations resulting in synergistic decreases in cellular viability.

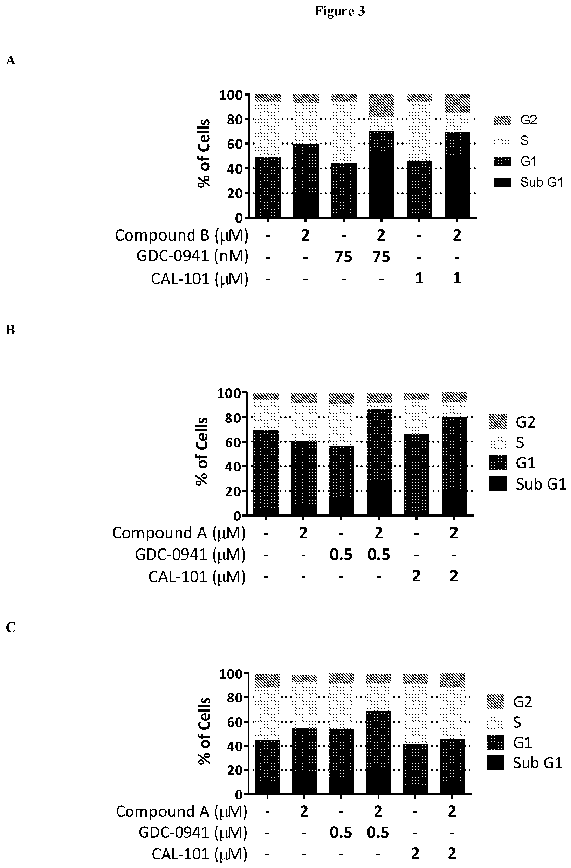

**Figures 3A-C** are graphs showing the effects of treatment of Mantle Cell lymphoma cells (Jeko1 - **Figure 3A****,** Mino - **Figure 3B****,** and Granta-519 - **Figure 3C**) with DMSO, **Compound A** or **B** (2 µM), GDC-0941 (0.075-0.5 µM), or the combination of **Compound A** or **B** (2 µM) with GDC-0941 (0.075-0.5 µM) on cell cycle inhibition. Alternatively, cells were treated with DMSO, **Compound A** or **B** (2 µM), CAL-101 (1-2 µM), or the combination of **Compound A** or **B** (2 µM) with CAL-101 (1-2 µM) before measuring effects on cell cycle inhibition. Combination treatment with either PI3K inhibitor resulted in further reductions in cell cycle progression consistent with decreased proliferation.

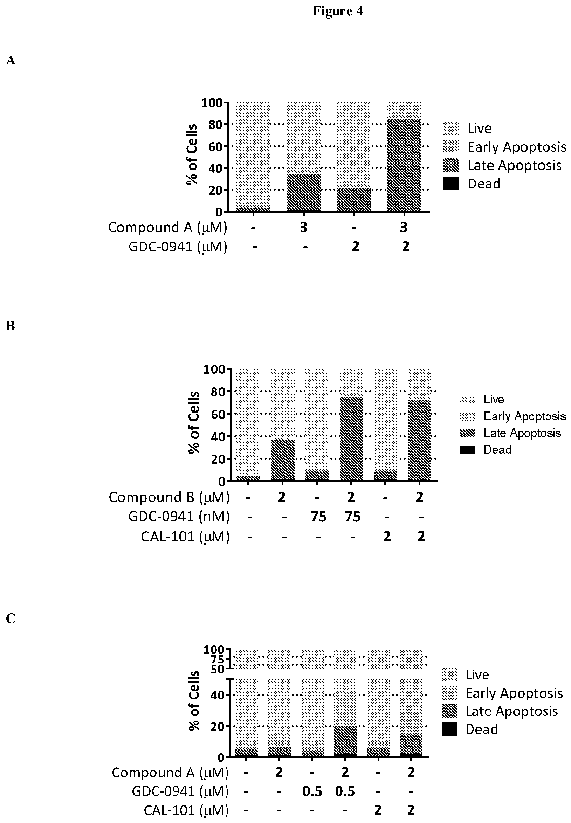

**Figures 4A-C** are graphs showing the effects of treatment of Jeko1 (**Figures 4A and 4B**) and Mino (**Figure 4C**) Mantle Cell lymphoma cells with DMSO, **Compound A** or **B** (2-3 µM), GDC-0941 (0.075-2 µM), or the combination of **Compound A** or **B** (2-3 µM) with GDC-0941 (0.075-2 µM) on the induction of apoptosis. Alternatively, cells were treated with DMSO, **Compound A** or **B** (2 µM), CAL-101 (2 µM), or the combination of **Compound A** or **B** (2 µM) with CAL-101 (2 µM) before measuring induction of apoptosis. Combination treatment with either PI3K inhibitor resulted in synergistic increases in cellular apoptosis.

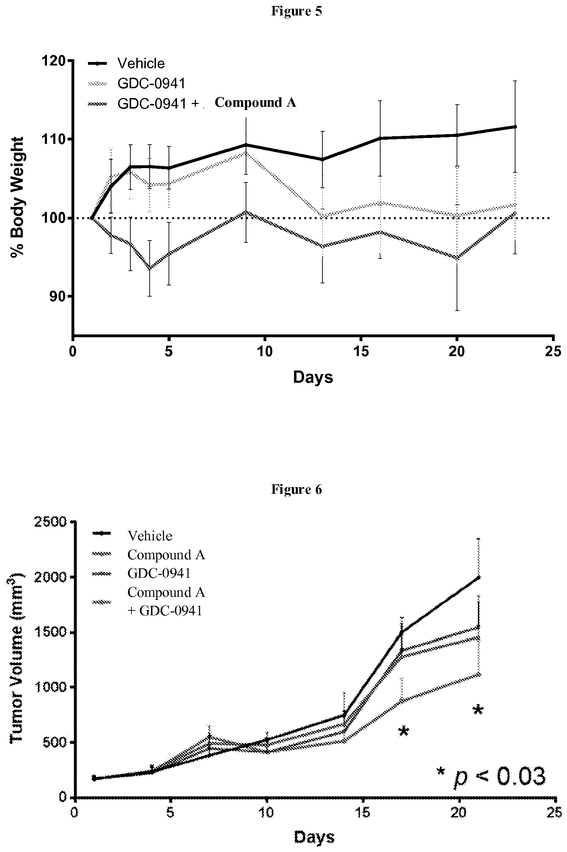

**Figure 5** is a graph showing the effects of treatment of CB-17 SCID mice with Vehicle, GDC-0941 alone (100 mg/kg PO QD), or GDC-0941 (100 mg/kg PO QD) plus **Compound A** (30 mg/kg IP QD) on body weight. All treatments were well tolerated with no overt evidence of toxicity, and the low level body weight loss was recovered by the end of the treatment period.
**Figure 6** is a graph showing significantly reduced tumor growth upon treatment of mice carrying Granta-519 tumor xenografts with the combination of **Compound A** (100 mg/kg PO BID) and GDC-0941 (75 mg/kg PO QD) relative to treatment with either single agent.

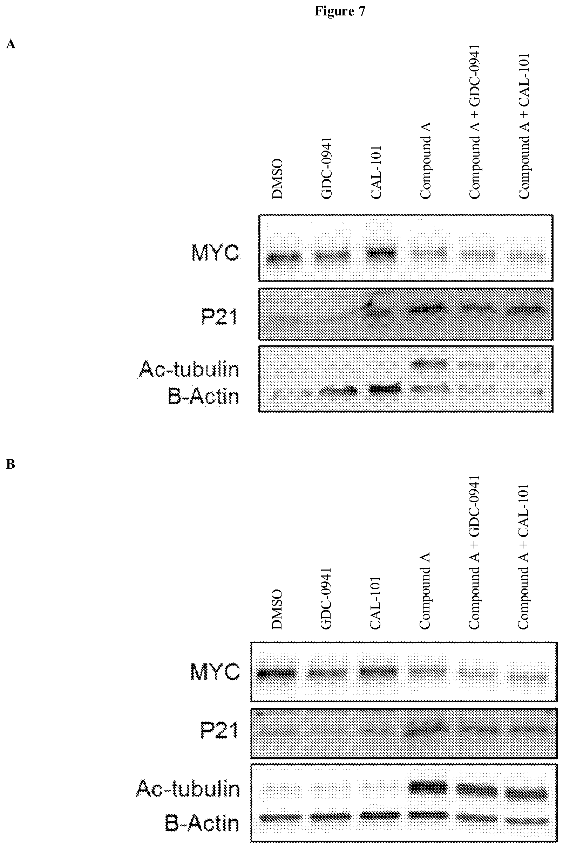

**Figures 7A-B** show images of immunoblots from Mino (**Figure 7A**) and Granta-519 (**Figure 7B**) cells showing that the combination of **Compound A** and either GDC-0941 or CAL-101 leads to further suppression of Myc expression, a key transcriptional regulator in cancer, relative to any of the single agents.

### DETAILED DESCRIPTION

The instant application is directed, generally, to combinations comprising a histone deacetylase (HDAC) inhibitor and a PI3K inhibitor as defined in the claims, for use in treating non-hodgkin's lymphoma.

### Definitions

Listed below are definitions of various terms used to describe this invention. These definitions apply to the terms as they are used throughout this specification and claims, unless otherwise limited in specific instances, either individually or as part of a larger group.

The term "about" generally indicates a possible variation of no more than 10%, 5%, or 1% of a value. For example, "about 25 mg/kg" will generally indicate, in its broadest sense, a value of 22.5-27.5 mg/kg, *i.e.,* 25 ± 2.5 mg/kg.

The term "alkyl" refers to saturated, straight- or branched-chain hydrocarbon moieties containing, in certain embodiments, between one and six, or one and eight carbon atoms, respectively. Examples of C₁-₆-alkyl moieties include methyl, ethyl, propyl, isopropyl, n-butyl, *tert*-butyl, neopentyl, n-hexyl moieties; and examples of C₁-₈-alkyl moieties include methyl, ethyl, propyl, isopropyl, *n*-butyl, *tert*-butyl, neopentyl, n-hexyl, heptyl, and octyl moieties.

The number of carbon atoms in an alkyl substituent can be indicated by the prefix "Cₓ-_{y}," where x is the minimum and y is the maximum number of carbon atoms in the substituent. Likewise, a Cₓ chain means an alkyl chain containing x carbon atoms.

The term "alkoxy" refers to an -O-alkyl moiety.

The terms "cycloalkyl" or "cycloalkylene" denote a monovalent group derived from a monocyclic or polycyclic saturated or partially unsaturated carbocyclic ring compound. Examples of C₃-₈-cycloalkyl include cyclopropyl, cyclobutyl, cyclopentyl, cyclohexyl, cycloheptyl and cyclooctyl; and examples of C₃-₁₂-cycloalkyl include cyclopropyl, cyclobutyl, cyclopentyl, cyclohexyl, bicyclo [2.2.1] heptyl, and bicyclo [2.2.2] octyl. Also contemplated are monovalent groups derived from a monocyclic or polycyclic carbocyclic ring compound having at least one carbon-carbon double bond by the removal of a single hydrogen atom. Examples of such groups include cyclopropenyl, cyclobutenyl, cyclopentenyl, cyclohexenyl, cycloheptenyl and cyclooctenyl.

The term "aryl" refers to a mono- or poly-cyclic carbocyclic ring system having one or more aromatic rings, fused or non-fused, including phenyl, naphthyl, tetrahydronaphthyl, indanyl and idenyl. In some embodiments, aryl groups have 6 carbon atoms. In some embodiments, aryl groups have from six to ten carbon atoms. In some embodiments, aryl groups have from six to sixteen carbon atoms.

The term "heteroaryl" refers to a mono- or poly-cyclic (*e.g.,* bi-, or tri-cyclic or more) fused or non-fused moiety or ring system having at least one aromatic ring, where one or more of the ring-forming atoms is a heteroatom such as oxygen, sulfur, or nitrogen. In some embodiments, the heteroaryl group has from about one to six carbon atoms, and in further embodiments from one to fifteen carbon atoms. In some embodiments, the heteroaryl group contains five to sixteen ring atoms of which one ring atom is selected from oxygen, sulfur, and nitrogen; zero, one, two, or three ring atoms are additional heteroatoms independently selected from oxygen, sulfur, and nitrogen; and the remaining ring atoms are carbon. Heteroaryl includes pyridinyl, pyrazinyl, pyrimidinyl, pyrrolyl, pyrazolyl, imidazolyl, thiazolyl, oxazolyl, isooxazolyl, thiazolyl, thiadiazolyl, oxadiazolyl, thiophenyl, furanyl, indolyl, quinolinyl, isoquinolinyl, benzimidazolyl, benzooxazolyl, quinoxalinyl and acridinyl.

The term "halo" refers to a halogen, such as fluorine, chlorine, bromine, and iodine.

The term "combination" refers to two or more therapeutic agents to treat a therapeutic condition or disorder described in the present disclosure. Such combination of therapeutic agents may be in the form of a single pill, capsule, or intravenous solution. However, the term "combination" also encompasses the situation when the two or more therapeutic agents are in separate pills, capsules, or intravenous solutions. Likewise, the term "combination therapy" refers to the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients or in multiple, or in separate containers (*e.g.,* capsules) for each active ingredient. In addition, such administration also encompasses use of each type of therapeutic agent in a sequential manner, either at approximately the same time or at different times. In either case, the treatment regimen will provide beneficial effects of the drug combination in treating the conditions or disorders described herein.

The term "HDAC" refers to histone deacetylases, which are enzymes that remove the acetyl groups from the lysine residues in core histones, thus leading to the formation of a condensed and transcriptionally silenced chromatin. There are currently 18 known histone deacetylases, which are classified into four groups. Class I HDACs, which include HDAC1, HDAC2, HDAC3, and HDAC8, are related to the yeast RPD3 gene. Class II HDACs, which include HDAC4, HDAC5, HDAC6, HDAC7, HDAC9, and HDAC10, are related to the yeast Hda1 gene. Class III HDACs, which are also known as the sirtuins are related to the Sir2 gene and include SIRT1-7. Class IV HDACs, which contains only HDAC11, has features of both Class I and II HDACs. The term "HDAC" refers to any one or more of the 18 known histone deacetylases, unless otherwise specified.

The term "HDAC6 specific" means that the compound binds to HDAC6 to a substantially greater extent, such as 5X, 10X, 15X, 20X greater or more, than to any other type of HDAC enzyme, such as HDAC1 or HDAC2. That is, the compound is selective for HDAC6 over any other type of HDAC enzyme. For example, a compound that binds to HDAC6 with an IC₅₀ of 10 nM and to HDAC1 with an IC₅₀ of 50 nM is HDAC6 specific. On the other hand, a compound that binds to HDAC6 with an IC₅₀ of 50 nM and to HDAC 1 with an IC₅₀ of 60 nM is not HDAC6 specific

The term "inhibitor" is synonymous with the term antagonist.

### Histone Deacetylase (HDAC) Inhibitors

Provided herein are pharmaceutical combinations for use in treating non-hodgkin's lymphoma in a subject in need thereof.

The combinations for use of the invention comprise a histone deacetylase (HDAC) inhibitor. The HDAC inhibitor is an HDAC6 specific inhibitor.

The HDAC6 specific inhibitor is selected from:

| | |
|---|---|
| 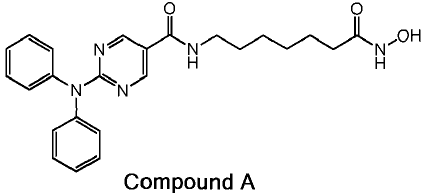 | 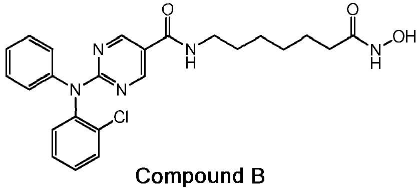 |
| 2-(diphenylamino)-N-(7-(hydroxyamino)-7-oxoheptyl)pyrimidine-5-carboxamide | 2-((2-chlorophenyl)(phenyl)amino)-N-(7-(hydroxyamino)-7-oxoheptyl)pyrimidine-5-carboxamide |
| IC₅₀(nM)HDAC6 = 10 HDAC3 = 84 | IC₅₀(nM) HDAC6 = 4 HDAC3 = 76 |

or pharmaceutically acceptable salts thereof.

The preparation and properties of selective HDAC6 inhibitors are provided in International Patent Application No. PCT/US2011/021982.

In some embodiments, the compounds described herein are unsolvated. In other embodiments, one or more of the compounds are in solvated form. As known in the art, the solvate can be any of pharmaceutically acceptable solvent, such as water and ethanol.

### Phosphoinositide 3-kinase (PI3K) Inhibitors

The combinations for use in the invention comprise a PI3K inhibitor selected from GDC-0941 and GS-1101.

A phosphatidyl-inositol 3-kinase (PI3K) inhibitor can be any compound or compounds capable of reducing a catalytic activity of any of the PI3K isoforms and subsequent PI3K-mediated signaling. In particular, PI3K inhibitors may be biological macromolecules or may be small organic or inorganic compounds. Preferably, the PI3K inhibitors are small organic compounds, and preferably synthetic compounds.

By "PI3K-mediated signaling" it is intended any of the biological activities that are dependent on, either directly or indirectly, the activity of PI3 kinase. Examples of PI3K-mediated signaling are signals that lead to proliferation and survival of PI3K-expressing cells, and stimulation of one or more PI3K-signaling pathways within PI3K-expressing cells.

A PI3K "signaling pathway" or "signal transduction pathway" means at least one biochemical reaction, or a group of biochemical reactions, that results from the activity of PI3K, and which generates a signal that, when transmitted through the signal pathway, leads to activation of one or more downstream molecules in the signaling cascade. Signal transduction pathways involve a number of signal transduction molecules that lead to transmission of a signal from the cell-surface across the plasma membrane of a cell, and through one or more in a series of signal transduction molecules, through the cytoplasm of the cell, and in some instances, into the cell's nucleus. Of particular interest to the present invention are PI3K signal transduction pathways which ultimately regulate (either enhance or inhibit) the activation of NF-κB via the NF-κB signaling pathway (see Hussain et al. PLoS One. 2012;7(6):e39945).

The PI3K inhibitors include CAL-101 and GDC-0941

The chemical structure of GDC-0941 is: 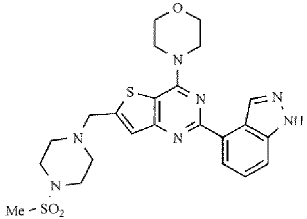 See Yao et al., "Suppression of Her2/Her3-mediated growth of breast cancer cells with combinations of GDC-0941 PI3K inhibitor, trastuzumab, and pertuzumab", Clin. Cancer Res., vol. 15(12), pp. 4147-56 (2009); Junttila et al., "Ligand-independent Her2/Her3/PI3K complex is disrupted by trastuzumab and is effectively inhibited by the PI3K inhibitor GDC-0941", Cancer Cell., vol. 15(5), pp. 429-440 (2009); and Folkes et al., "The identification of 2-(1H-indazol-4-yl)-6-(4-methanesulfonyl-piperazin-1-ylmehtyl)-4-morpholin-4-yl-thienol[3,2-d]pyrimidine (GDC-0941) as a potent, selective, orally bioavailable inhibitor of class I PI3 kinase for the treatment of cancer", J. Med. Chem., vol. 51(18), pp. 5522-32 (2008).

The chemical structure of GS-1101/CAL-101 is: 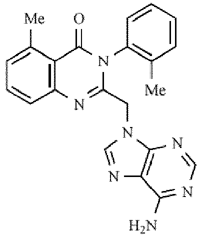 See U.S. patent numbers: 6800620; 6949535; 8138195; 8492389; 8637533; RE44599; and RE44638.

In some embodiments, the compounds described herein are unsolvated. In other embodiments, one or more of the compounds are in solvated form. As known in the art, the solvate can be any of pharmaceutically acceptable solvent, such as water, ethanol, and the like.

### Compositions, Combinations, and Pharmaceutical Compositions and Combinations

Provided herein are combinations for use in treating non-hodgkin's lymphoma in a subject in need thereof. Provided are combinations comprising a histone deacetylase (HDAC) inhibitor and a phosphatidylinositide 3-kinase (PI3K) inhibitor as defined in the claims, for use in the treatment of non-hodgkin's lymphoma in a subject in need thereof.

The HDAC inhibitor is an HDAC6 specific inhibitor. In specific embodiments, the HDAC6 specific inhibitor is: 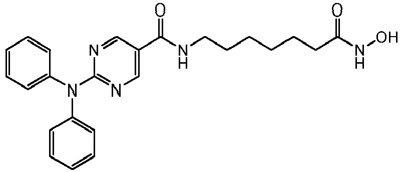 or a pharmaceutically acceptable salt thereof.

In yet other embodiments, the HDAC6 specific inhibitor is: 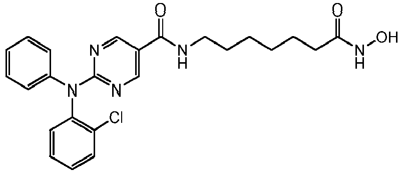 or a pharmaceutically acceptable salt thereof.

The phosphatidylinositide 3-kinase (PI3K) inhibitor is GDC-0941 or GS-1101, or a pharmaceutically acceptable salt thereof.

In one embodiment, provided herein is a combination therapy comprising an HDAC6 specific inhibitor and a phosphatidylinositide 3-kinase (PI3K) inhibitor, wherein the HDAC6 specific inhibitor is 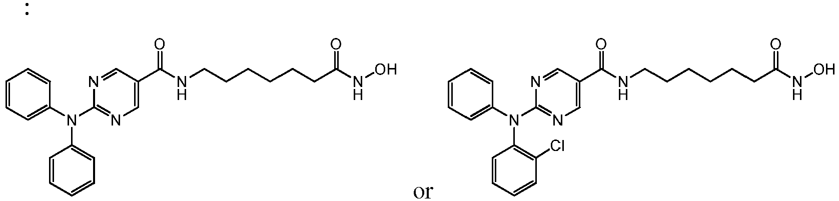 or pharmaceutically acceptable salts thereof; and
the PI3K inhibitor is GDC-0941 or GS-1101, or a pharmaceutically acceptable salt thereof.

Although the HDAC6 specific inhibitors are depicted in their neutral forms, in some embodiments, these compounds are used in a pharmaceutically acceptable salt form. "Pharmaceutically acceptable salts" refers to derivatives of the disclosed compounds wherein the parent compound is modified by converting an existing acid or base moiety to its salt form. Examples of pharmaceutically acceptable salts include mineral or organic acid salts of basic residues such as amines; alkali or organic salts of acidic residues such as carboxylic acids. The pharmaceutically acceptable salts of the present invention include the conventional non-toxic salts of the parent compound formed, for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17.sup.th ed., Mack Publishing Company, Easton, Pa., 1985, p. 1418 and Journal of Pharmaceutical Science, 66, 2 (1977).

### Administration/Dose

In some embodiments, the HDAC inhibitor is administered simultaneously with the phosphatidylinositide 3-kinase (PI3K) inhibitor. Simultaneous administration typically means that both compounds enter the patient at precisely the same time. However, simultaneous administration also includes the possibility that the HDAC inhibitor and the phosphatidylinositide 3-kinase (PI3K) inhibitor enter the patient at different times, but the difference in time is sufficiently miniscule that the first administered compound is not provided the time to take effect on the patient before entry of the second administered compound. Such delayed times typically correspond to less than 1 minute, and more typically, less than 30 seconds. In one example, wherein the compounds are in solution, simultaneous administration can be achieved by administering a solution containing the combination of compounds. In another example, simultaneous administration of separate solutions, one of which contains the HDAC inhibitor and the other of which contains the phosphatidylinositide 3-kinase (PI3K) inhibitor, can be employed. In one example wherein the compounds are in solid form, simultaneous administration can be achieved by administering a composition containing the combination of compounds. Alternatively, simultaneous administration can be achieved by administering two separate compositions, one comprising the HDAC inhibitor and the other comprising the phosphatidylinositide 3-kinase (PI3K) inhibitor.

In other embodiments, the HDAC inhibitor and the phosphatidylinositide 3-kinase (PI3K) inhibitor are not administered simultaneously. In some embodiments, the HDAC inhibitor is administered before the phosphatidylinositide 3-kinase (PI3K) inhibitor. In other embodiments, the phosphatidylinositide 3-kinase (PI3K) inhibitor is administered before the HDAC inhibitor. The time difference in non-simultaneous administrations can be greater than 1 minute, five minutes, 10 minutes, 15 minutes, 30 minutes, 45 minutes, 60 minutes, two hours, three hours, six hours, nine hours, 12 hours, etc. In other embodiments, the first administered compound is provided time to take effect on the patient before the second administered compound is administered. Generally, the difference in time does not extend beyond the time for the first administered compound to complete its effect in the patient, or beyond the time the first administered compound is completely or substantially eliminated or deactivated in the patient.

In some embodiments, one or both of the HDAC inhibitor and the phosphatidylinositide 3-kinase (PI3K) inhibitor are administered in a therapeutically effective amount or dosage. A "therapeutically effective amount" is an amount of HDAC6 specific inhibitor or a phosphatidylinositide 3-kinase (PI3K) inhibitor that, when administered to a patient by itself, effectively treats the non-hodgkin's lymphoma. An amount that proves to be a "therapeutically effective amount" in a given instance, for a particular subject, may not be effective for 100% of subjects similarly treated for the disease or condition under consideration, even though such dosage is deemed a "therapeutically effective amount" by skilled practitioners. The amount of the compound that corresponds to a therapeutically effective amount is strongly dependent on the type of cancer, stage of the cancer, the age of the patient being treated, and other facts. In general, therapeutically effective amounts of these compounds are well-known in the art, such as provided in the supporting references cited above.

In other embodiments, one or both of the HDAC inhibitor and the phosphatidylinositide 3-kinase (PI3K) inhibitor are administered in a sub-therapeutically effective amount or dosage. A sub-therapeutically effective amount is an amount of HDAC inhibitor or a phosphatidylinositide 3-kinase (PI3K) inhibitor that, when administered to a patient by itself, does not completely inhibit over time the biological activity of the intended target.

Whether administered in therapeutic or sub-therapeutic amounts, the combination of the HDAC inhibitor and the phosphatidylinositide 3-kinase (PI3K) inhibitor should be effective in treating non-hodgkin's lymphoma. For example, a sub-therapeutic amount of the phosphatidylinositide 3-kinase (PI3K) inhibitor can be an effective amount if, when combined with a HDAC6 specific inhibitor, the combination is effective in the treatment of non-hodgkin's lymphoma.

In some embodiments, the combination of compounds exhibits a synergistic effect (*i.e.,* greater than additive effect) in the treatment of the non-hodgkin's lymphoma. The term "synergistic effect" refers to the action of two agents, such as, for example, a HDAC inhibitor and a phosphatidylinositide 3-kinase (PI3K) inhibitor, producing an effect, for example, slowing the symptomatic progression of cancer or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

In different embodiments, depending on the combination and the effective amounts used, the combination of compounds can inhibit cancer growth, achieve cancer stasis, or even achieve substantial or complete cancer regression.

While the amounts of a HDAC inhibitor and a phosphatidylinositide 3-kinase (PI3K) inhibitor should result in the effective treatment of the non-hodgkin's lymphoma, the amounts, when combined, are preferably not excessively toxic to the patient (*i.e.,* the amounts are preferably within toxicity limits as established by medical guidelines). In some embodiments, either to prevent excessive toxicity and/or provide a more efficacious treatment of the non-hodgkin's lymphoma, a limitation on the total administered dosage is provided. Typically, the amounts considered herein are per day; however, half-day and two-day or three-day cycles also are considered herein.

Different dosage regimens may be used to treat the non-hodgkin's lymphoma. In some embodiments, a daily dosage, such as any of the exemplary dosages described above, is administered once, twice, three times, or four times a day for three, four, five, six, seven, eight, nine, or ten days. Depending on the stage and severity of the non-hodgkin's lymphoma, a shorter treatment time (*e.g.,* up to five days) may be employed along with a high dosage, or a longer treatment time (*e.g.,* ten or more days, or weeks, or a month, or longer) may be employed along with a low dosage. In some embodiments, a once- or twice-daily dosage is administered every other day.

In some embodiments, each dosage contains both an HDAC inhibitor and a phosphatidylinositide 3-kinase (PI3K) inhibitor to be delivered as a single dosage, while in other embodiments, each dosage contains either a HDAC inhibitor and a phosphatidylinositide 3-kinase (PI3K) inhibitor to be delivered as separate dosages.

The HDAC6 specific inhibitors, or their pharmaceutically acceptable salts or solvate forms, in pure form or in an appropriate pharmaceutical composition, can be administered via any of the accepted modes of administration or agents known in the art. The compounds can be administered, for example, orally, nasally, parenterally (intravenous, intramuscular, or subcutaneous), topically, transdermally, intravaginally, intravesically, intracistemally, or rectally. The dosage form can be, for example, a solid, semi-solid, lyophilized powder, or liquid dosage forms, such as for example, tablets, pills, soft elastic or hard gelatin capsules, powders, solutions, suspensions, suppositories, aerosols, or the like, preferably in unit dosage forms suitable for simple administration of precise dosages. A particular route of administration is oral, particularly one in which a convenient daily dosage regimen can be adjusted according to the degree of severity of the disease to be treated.

As discussed above, the HDAC inhibitor and the PI3K inhibitor of the pharmaceutical combination can be administered in a single unit dose or separate dosage forms. Accordingly, the phrase "pharmaceutical combination" includes a combination of two drugs in either a single dosage form or a separate dosage forms, *i.e.,* the pharmaceutically acceptable carriers and excipients described throughout the application can be combined with an HDAC inhibitor and a PI3K inhibitor in a single unit dose, as well as individually combined with a HDAC inhibitor and a PI3K inhibitor when these compounds are administered separately.

Auxiliary and adjuvant agents may include, for example, preserving, wetting, suspending, sweetening, flavoring, perfuming, emulsifying, and dispensing agents. Prevention of the action of microorganisms is generally provided by various antibacterial and antifungal agents, such as, parabens, chlorobutanol, phenol and sorbic acid. Isotonic agents, such as sugars and sodium chloride, may also be included. Prolonged absorption of an injectable pharmaceutical form can be brought about by the use of agents delaying absorption, for example, aluminum monostearate and gelatin. The auxiliary agents also can include wetting agents, emulsifying agents, pH buffering agents, and antioxidants, such as, for example, citric acid, sorbitan monolaurate, triethanolamine oleate and butylated hydroxytoluene.

Solid dosage forms can be prepared with coatings and shells, such as enteric coatings and others well-known in the art. They can contain pacifying agents and can be of such composition that they release the active compound or compounds in a certain part of the intestinal tract in a delayed manner. Examples of embedded compositions that can be used are polymeric substances and waxes. The active compounds also can be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., the HDAC inhibitors or phosphatidylinositide 3-kinase (PI3K) inhibitors described herein, or a pharmaceutically acceptable salt thereof, and optional pharmaceutical adjuvants in a carrier, such as, for example, water, saline, aqueous dextrose, glycerol and ethanol; solubilizing agents and emulsifiers, as for example, ethyl alcohol, isopropyl alcohol, ethyl carbonate, ethyl acetate, benzyl alcohol, benzyl benzoate, propyleneglycol, 1,3- butyleneglycol, dimethyl formamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, to thereby form a solution or suspension.

Generally, depending on the intended mode of administration, the pharmaceutically acceptable compositions will contain about 1% to about 99% by weight of the compounds described herein, or a pharmaceutically acceptable salt thereof, and 99% to 1% by weight of a pharmaceutically acceptable excipient. In one example, the composition will be between about 5% and about 75% by weight of a compound described herein, or a pharmaceutically acceptable salt thereof, with the rest being suitable pharmaceutical excipients.

Actual methods of preparing such dosage forms are known, or will be apparent, to those skilled in this art. Reference is made, for example, to Remington's Pharmaceutical Sciences, 18th Ed., (Mack Publishing Company, Easton, Pa., 1990).

There are provided methods for treating non-hodgkin's lymphoma in a subject in need thereof comprising administering to the subject a pharmaceutical combination of the invention. Thus, provided herein are methods for treating non-hodgkin's lymphoma in a subject in need thereof comprising administering to the subject a a combination comprising an HDAC inhibitor and a phosphatidylinositide 3-kinase (PI3K) inhibitor.

The subject considered herein is typically a human. However, the subject can be any mammal for which treatment is desired. Thus, the methods described herein can be applied to both human and veterinary applications.

The terms "treating" or "treatment" indicate that the method has, at the least, mitigated abnormal cellular proliferation. For example, the method can reduce the rate of cellular growth in a patient, or prevent the continued growth or spread of non-hodgkin's lymphoma, or even reduce the overall reach of the non-hodgkin's lymphoma. Inhibition of abnormal cell growth can occur by a variety of mechanisms including cell death, apoptosis, arrest of mitosis, inhibition of cell division, transcription, translation, transduction, etc.

### Kits

There are also provided kits. In some embodiments, kits comprise a HDAC inhibitor or a pharmaceutically acceptable salt thereof and a phosphatidylinositide 3-kinase (PI3K) inhibitor or a pharmaceutically acceptable salt thereof.

The phrase "package" means any vessel containing compounds or compositions presented herein. In some embodiments, the package can be a box or wrapping. Packaging materials for use in packaging pharmaceutical products are well-known to those of skill in the art. Examples of pharmaceutical packaging materials include bottles, tubes, inhalers, pumps, bags, vials, containers, syringes, bottles, and any packaging material suitable for a selected formulation and intended mode of administration and treatment.

The kit can also contain items that are not contained within the package, but are attached to the outside of the package, for example, pipettes.

Kits can further contain instructions for administering compounds or compositions of the invention to a patient. Kits also can comprise instructions for approved uses of compounds herein by regulatory agencies, such as the United States Food and Drug Administration. Kits can also contain labeling or product inserts for the compounds. The package(s) and/or any product insert(s) may themselves be approved by regulatory agencies. The kits can include compounds in the solid phase or in a liquid phase (such as buffers provided) in a package. The kits can also include buffers for preparing solutions for conducting the methods, and pipettes for transferring liquids from one container to another.

### EXAMPLES

Examples have been set forth below for the purpose of illustration and to describe certain specific embodiments of the invention. However, the scope of the claims is not to be in any way limited by the examples set forth herein.

The synthesis of the compounds of Formula I (e.g., Compounds A and B) is provided in PCT/US2011/021982. The synthesis of compounds of Formula II (e.g., Compounds C and D) is provided in PCT/US2011/060791.

### Example 1: Synthesis of 2-(diphenylamino)-N-(7-(hydroxyamino)-7-oxoheptyl) pyrimidine-5-carboxamide (Compound A)

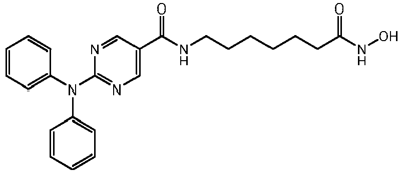

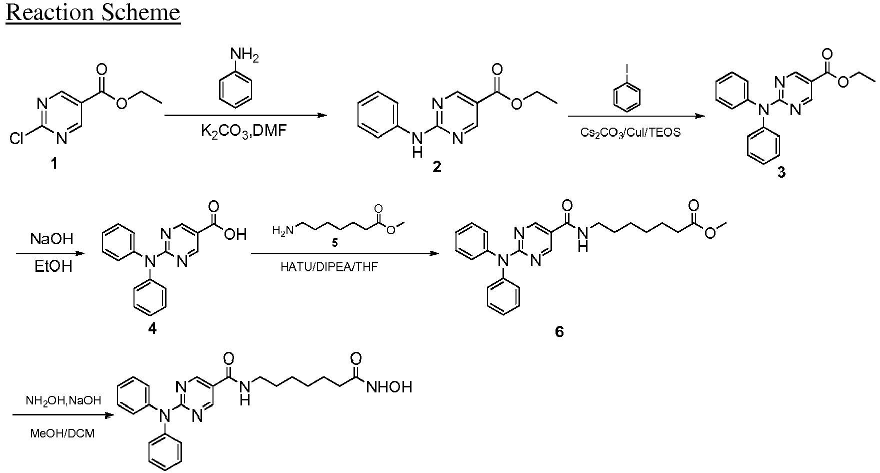

Synthesis of Intermediate 2: A mixture of aniline (3.7 g, 40 mmol), compound 1 (7.5 g, 40 mmol), and K₂CO₃ (11 g, 80 mmol) in DMF (100 ml) was degassed and stirred at 120 ° C under N₂ overnight. The reaction mixture was cooled to r.t. and diluted with EtOAc (200 ml), then washed with saturated brine (200 ml × 3). The organic layers were separated and dried over Na₂SO₄, evaporated to dryness and purified by silica gel chromatography (petroleum ethers/EtOAc = 10/1) to give the desired product as a white solid (6.2 g, 64 %).

Synthesis of Intermediate 3: A mixture of compound **2** (6.2 g, 25 mmol), iodobenzene (6.12 g, 30 mmol), CuI (955 mg, 5.0 mmol), Cs₂CO₃ (16.3 g, 50 mmol) in TEOS (200 ml) was degassed and purged with nitrogen. The resulting mixture was stirred at 140 ° C for 14 hrs. After cooling to r.t., the residue was diluted with EtOAc (200 ml). 95% EtOH (200 ml) and NH₄F-H₂O on silica gel [50g, pre-prepared by the addition of NH₄F (100g) in water (1500 ml) to silica gel (500g, 100-200 mesh)] was added, and the resulting mixture was kept at r.t. for 2 hrs. The solidified materials were filtered and washed with EtOAc. The filtrate was evaporated to dryness and the residue was purified by silica gel chromatography (petroleum ethers/EtOAc = 10/1) to give a yellow solid (3 g, 38%).

Synthesis of Intermediate 4: 2N NaOH (200 ml) was added to a solution of compound **3** (3.0 g, 9.4 mmol) in EtOH (200 ml). The mixture was stirred at 60 ° C for 30min. After evaporation of the solvent, the solution was neutralized with 2N HCl to give a white precipitate. The suspension was extracted with EtOAc (2 × 200 ml), and the organic layers were separated, washed with water (2 × 100 ml), brine (2 × 100 ml), and dried over Na₂SO₄. Removal of the solvent gave a brown solid (2.5 g, 92 %).

Synthesis of Intermediate 6: A mixture of compound **4** (2.5 g, 8.58 mmol), compound **5** (2.52 g, 12.87 mmol), HATU (3.91 g, 10.30 mmol), and DIPEA (4.43 g, 34.32 mmol) was stirred at r.t. overnight. After the reaction mixture was filtered, the filtrate was evaporated to dryness and the residue was purified by silica gel chromatography (petroleum ethers/EtOAc = 2/1) to give a brown solid (2 g, 54 %).

Synthesis of 2-(diphenylamino)-N-(7-(hydroxyamino)-7-oxoheptyl)pyrimidine-5-carboxamide (**Compound A**): A mixture of the compound **6** (2.0 g, 4.6 mmol), sodium hydroxide (2N, 20 mL) in MeOH (50 ml) and DCM (25 ml) was stirred at 0 ° C for 10 min. Hydroxylamine (50%) (10 ml) was cooled to 0 ° C and added to the mixture. The resulting mixture was stirred at r.t. for 20 min. After removal of the solvent, the mixture was neutralized with 1M HCl to give a white precipitate. The crude product was filtered and purified by pre-HPLC to give a white solid (950 mg, 48%).

### Example 2: Synthesis of 2-((2-chlorophenyl)(phenyl)amino)-N-(7-(hydroxyamino)-7-oxoheptyl)pyrimidine-5-carboxamide (Compound B)

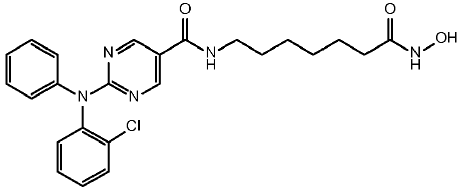

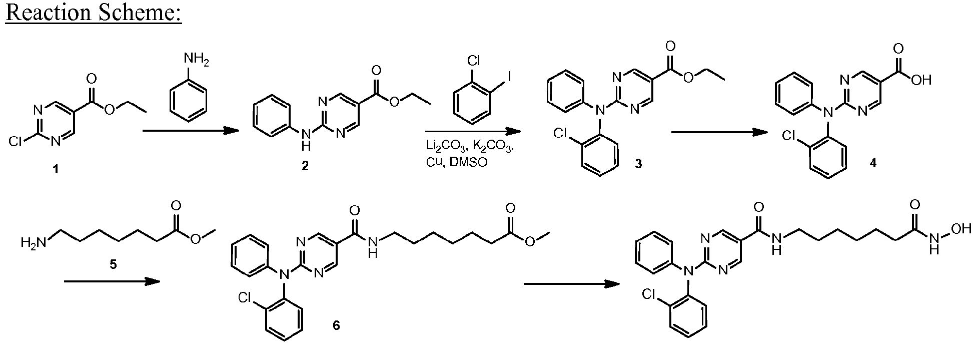

Synthesis of Intermediate 2: See synthesis of intermediate 2 in Example 1.

Synthesis of Intermediate 3: A mixture of compound 2 (69.2 g, 1 equiv.), 1-chloro-2-iodobenzene (135.7 g, 2 equiv.), Li₂CO₃ (42.04 g, 2 equiv.), K₂CO₃ (39.32 g, 1 equiv.), Cu (1 equiv. 45 µm) in DMSO (690 ml) was degassed and purged with nitrogen. The resulting mixture was stirred at 140 ° C. Work-up of the reaction gave compound 3 at 93 % yield.

Synthesis of Intermediate 4: See synthesis of intermediate 4 in Example 1.

Synthesis of Intermediate 6: See synthesis of intermediate 6 in Example 1.

Synthesis of 2-((2-chlorophenyl)(phenyl)amino)-N-(7-(hydroxyamino)-7-oxoheptyl)pyrimidine-5-carboxamide (**Compound B**): See synthesis of **Compound A** in Example 1.

### Example 3*: Synthesis of 2-((1-(3-fluorophenyl)cyclohexyl)amino)-N-hydroxypyrimidine-5-carboxamide (Compound C)

***This example is not within the scope of currently claimed invention**

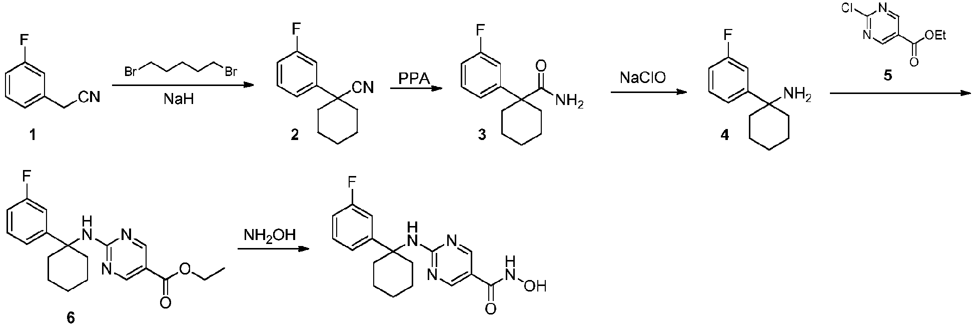

Synthesis of Intermediate 2: To a solution of compound 1 (100 g, 0.74 mol) in dry DMF (1000 ml) was added 1,5-dibromopentane (170 g, 0.74 mol). NaH (65 g, 2.2 eq) was added dropwise while the reaction was cooled in an ice bath. The resulting mixture was vigorously stirred overnight at 50 °C. The suspension was carefully quenched with ice water and extracted with ethyl acetate (3 × 500 ml). The combined organic layers were concentrated to afford the crude product, which was purified by flash column chromatography to give compound **2** as pale solid (100 g, 67%).

Synthesis of Intermediate 3: A solution of compound **2** (100 g, 0.49 mol) in PPA (500 ml) was heated at 110 °C for about 5-6 hours. After completion, the resulting mixture was carefully adjusted to a pH of about 8-9 with sat.NaHCO₃ solution. The resulting precipitate was collected and washed with water (1000 ml) to afford compound **3** as white solid (95 g, 87%).

Synthesis of Intermediate 4: To a solution of compound **3** (95 g, 0.43 mol) in n-BuOH (800 ml) was added NaClO (260 ml, 1.4 eq). 3N NaOH (400 ml, 2.8 equiv.) was then added at 0 °C and the reaction was stirred overnight at r.t. The resulting mixture was extracted with EA (2 × 500 ml), and the combined organic layers washed with brine. The solvent was removed in vacuo to afford the crude product which was further purified by treatment with HCl salt to yield compound **4** as a white powder (72 g, 73%).

Synthesis of Intermediate 6: To a solution of compound **4** (2.29 g 10 mmol) in dioxane (50 ml) was added compound **5** (1.87 g, 1.0 equiv.) and DIPEA (2.58 g, 2.0 equiv.). The mixture was heated overnight at 110-120 °C. The resulting mixture was directly purified on silica gel column to afford the coupled product, compound **6,** as a white solid (1.37 g, 40%).

### Synthesis of 2-((1-(3-fluorophenyl)cyclohexyl)amino)-N-hydroxypyrimidine-5-carboxamide (Compound C):

To a solution of compound **6** (100 mg, 0.29 mmol) in MeOH/DCM(10 ml, 1:1) was added 50% NH₂OH in water (2 ml, excess). Sat. NaOH in MeOH (2 ml, excess) was then added at 0 °C and the reaction was stirred for 3-4 hours. After completion, the resulting mixture was concentrated and acidified with 2N HCl to reach a pH of 4-5. The precipitate was collected and washed with water (10 ml) to remove excess NH₂OH. Drying the precipitate afforded 2-((1-(3-fluorophenyl)cyclohexyl)amino)-N-hydroxypyrimidine-5-carboxamide as a white powder (70 mg, 73%).

### Example 4*: Synthesis of N-hydroxy-2-((1-phenylcyclopropyl)amino)pyrimidine-5-carboxamide (Compound D)

***This example is not within the scope of currently claimed invention**

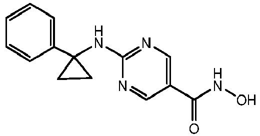

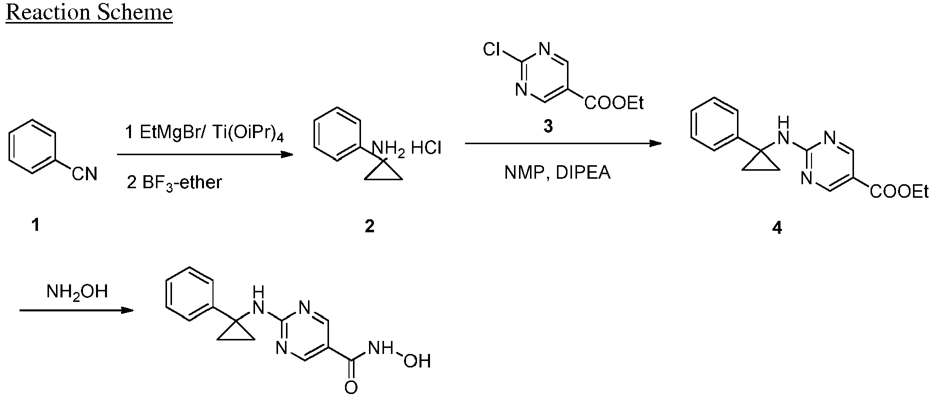

Synthesis of Intermediate 2: A solution of compound 1, benzonitrile, (250 g, 1.0 equiv.), and Ti(OiPr)₄ (1330 ml, 1.5 equiv.) in MBTE (3750 ml) was cooled to about -10 to -5 °C under a nitrogen atmosphere. EtMgBr (1610 ml, 3.0M, 2.3 equiv.) was added dropwise over a period of 60 min., during which the inner temperature of the reaction was kept below 5 °C. The reaction mixture was allowed to warm to 15-20 °C for 1 hr. BF₃-ether (1300 ml, 2.0 equiv.) was added dropwise over a period of 60 min., while the inner temperature was maintained below 15 °C. The reaction mixture was stirred at 15-20 °C for 1-2 hr. and stopped when a low level of benzonitrile remained. 1N HCl (2500 ml) was added dropwise while maintaining the inner temperature below 30 °C. NaOH (20%, 3000 ml) was added dropwise to bring the pH to about 9.0, while still maintaining a temperature below 30 °C. The reaction mixture was extracted with MTBE (3 L × 2) and EtOAc (3 L × 2), and the combined organic layers were dried with anhydrous Na₂SO₄ and concentrated under reduced pressure (below 45 °C) to yield a red oil. MTBE (2500 ml) was added to the oil to give a clear solution, and upon bubbling with dry HCl gas, a solid precipitated. This solid was filtered and dried in vacuum yielding 143 g of compound **2.**

Synthesis of Intermediate 4: Compound **2** (620 g, 1.0 equiv) and DIPEA (1080 g, 2.2 equiv. were dissolved in NMP (3100 ml) and stirred for 20 min. Compound **3** (680 g, 1.02 equiv.) was added and the reaction mixture was heated to about 85-95 °C for 4 hrs. The solution was allowed to slowly cool to r.t. This solution was poured onto H₂O (20 L) and much of the solid was precipitated out from the solution with strong stirring. The mixture was filtered and the cake was dried under reduced pressure at 50 °C for 24 hr., yielding 896 g of compound **4** (solid, 86.8%).

Synthesis of N-hydroxy-2-((1-phenylcyclopropyl)amino)pyrimidine-5-carboxamide (**Compound D**): A solution of MeOH(1000 ml) was cooled to about 0-5 °C with stirring. NH₂OH HCl (1107 g, 10 equiv.) was added, followed by careful addition of NaOCH₃ (1000 g, 12.0 equiv.) The resulting mixture was stirred at 0-5 °C for one hr, and was filtered to remove the solid. Compound **4** (450 g, 1.0 equiv.) was added to the reaction mixture in one portion, and stirred at 10 °C for two hours until compound **4** was consumed. The reaction mixture was adjusted to a pH of about 8.5-9 through addition of HCl (6N), resulting in precipitation. The mixture was concentrated under reduced pressure. Water (3000 ml) was added to the residue with intense stirring and the precipitate was collected by filtration. The product was dried in an oven at 45 °C overnight (340 g, 79% yield).

### Example 5: HDAC Enzyme Assays

Compounds for testing were diluted in DMSO to 50 fold the final concentration and a ten point three fold dilution series was made. The compounds were diluted in assay buffer (50 mM HEPES, pH 7.4, 100 mM KCl, 0.001% Tween-20, 0.05% BSA, 20 µM TCEP) to 6 fold their final concentration. The HDAC enzymes (purchased from BPS Biosciences) were diluted to 1.5 fold their final concentration in assay buffer. The tripeptide substrate and trypsin at 0.05 µM final concentration were diluted in assay buffer at 6 fold their final concentration. The final enzyme concentrations used in these assays were 3.3 ng/ml (HDAC1), 0.2 ng/ml (HDAC2), 0.08 ng/ml (HDAC3) and 2 ng/ml (HDAC6). The final substrate concentrations used were 16 µM (HDAC1), 10 µM (HDAC2), 17 µM (HDAC3) and 14 µM (HDAC6). Five µl of compound and 20 µl of enzyme were added to wells of a black, opaque 384 well plate in duplicate. Enzyme and compound were incubated together at room temperature for 10 minutes. Five µl of substrate was added to each well, the plate was shaken for 60 seconds and placed into a Victor 2 microtiter plate reader. The development of fluorescence was monitored for 60 min and the linear rate of the reaction was calculated. The IC50 was determined using Graph Pad Prism by a four parameter curve fit.

### Example 6: Inhibition of HDAC6 in a Collection of NHL Cell Lines, Both as a Single Agent and in Combination

This example describes the therapeutic potential of inhibiting HDAC6 in a collection of NHL cell lines, both as a single agent and in combination with these novel targeted agents. Treatment of lymphoma cells from a variety of molecular subtypes with selective HDAC6 inhibitors, including **Compound A, Compound B,** and the highly selective **Compound C*,** in combination with an inhibitor of PI3K resulted in synergistic decreases in lymphoma cell viability.

Human lymphoma cell lines were selected that represented the most common subtypes of NHL. For Mantle Cell Lymphoma (MCL) Mino, Jeko1, and Granta-519 cells were utilized, while U2932 and SUDHL16 cells represented the activated B cell (ABC) and germinal center (GC) subtypes of diffuse large B cell lymphoma (DLBCL), respectively. Briefly, cells were seeded in 384-well plates and treated in quadruplicate in a dose-matrix format with an HDAC6 inhibitor (**Compound A, Compound B,** or **Compound C***) in combination with a PI3K inhibitor (GDC-0941 or GS-1101/CAL-101). After incubating these cells for 48hr, total cell viability was assessed via an MTS assay (Aqueous One, Promega). The fraction affected (Fa) was subsequently determined for each dose combination and the combination index (CI) was assessed using the method of Chou-Talay. CI values less than one represent a synergistic effect, values equal to one suggest an additive effect, and values greater than two indicate an antagonistic effect. As can be seen in the Fa-CI plots in **Figures 1A-F** and **Figures 2A-2F****,** in all five lymphoma cell lines the HDAC6 inhibitors showed strong evidence of synergy with both PI3K inhibitors tested. This is evidenced by the large number of data points (representing individual dose combinations) in the Fa-CI plot that fall below the highly stringent cutoff of 0.7. Together, these results provide strong evidence that inhibition of HDAC6 in combination with inhibition of PI3K results in synergistic cell killing, and further suggests that combinations of drugs targeting both HDAC6 and PI3K may provide significant clinical benefit for NHL patients.

***Not within the scope of currently claimed invention**

### Example 7: The Combination of an HDAC6 Inhibitor and a PI3K Inhibitor Affects Cellular Proliferation and Cell Cycle Progression

This example provides evidence of how the combination of an HDAC6 inhibitor and a PI3K inhibitor affects cellular proliferation and cell cycle progression. Treatment of lymphoma cells with **Compound A, Compound B,** and/or PI3K inhibitor resulted in decreased cell cycle progression, indicative of decreased proliferation.

Jeko1 (**Figure 3A**), Mino (**Figure 3B**) or Granta-519 (**Figure 3C**) lymphoma cells were exposed to drug for 4 days, and cell cycle distribution was assessed by flow cytometry via incorporation of EdU (5-ethynyl-2'-deoxyuridine) and staining with propidium iodide. The relative fraction of cells in each stage of the cell cycle (G0/G1, S, and G2/M), as well as the fraction of dead cells (Sub G1) was then estimated. Cells were treated with DMSO, **Compound A** or **B** (2 µM), GDC-0941 (0.075-0.5 µM), or the combination of **Compound A** or **B** (2 µM) with GDC-0941 (0.075-0.5 µM) on cell cycle inhibition. Alternatively, cells were treated with DMSO, **Compound A** or **B** (2 µM), CAL-101 (1-2 µM), or the combination of **Compound A** or **B** (2 µM) with CAL-101 (1-2 µM) before measuring effects on cell cycle inhibition.

In all cells, combination treatment resulted in a significant decrease in the percentage of cells actively proliferating in the S phase of the cell cycle, consistent with reduced proliferation in these cells. Combination treatment also resulted in an increase in the Sub G1 population in both cell lines, consistent with increased cell death as a result of the combination treatment with an HDAC6i and a PI3Ki.

### Example 8: The Combination of an HDAC6 Inhibitor and a PI3K Inhibitor Induces Apoptosis in Lymphoma Cells

This example provides evidence of how the combination of an HDAC6 inhibitor and a PI3K inhibitor induces apoptosis in lymphoma cells. Treatment of lymphoma cells with **Compound A or B** plus a PI3Ki resulted in synergistic increases in cellular apoptosis.

Jeko1 (**Figure 4A** and **Figure 4B**) or Mino (**Figure 4C**) lymphoma cells were exposed to drug for 4 days, and apoptosis was assessed by flow cytometry by measuring Annexin V binding and cellular permeability to propidium iodide. The relative fraction of cells that were live, in early apoptosis, in late apoptosis, or dead was then estimated. The cells were treated with DMSO, **Compound A** or **B** (2-3 µM), GDC-0941 (0.075-2 µM), or the combination of **Compound A** or **B** (2-3 µM) with GDC-0941 (0.075-2 µM) on the induction of apoptosis. Alternatively, cells were treated with DMSO, **Compound A** or **B** (2 µM), CAL-101 (2 µM), or the combination of **Compound A** or **B** (2 µM) with CAL-101 (2 µM) before measuring induction of apoptosis.

Treatment with **Compound A or B** resulted in a small increase in apoptosis relative to control cells, while treatment with GDC-0941 or CAL-101 did not result in increased cell death. However, the combination of **Compound** A **or B** with GDC-0941 or CAL-101 resulted in synergistic increases in the percentage of apoptotic cells.

### Example 9: The Combination of an HDAC6 Inhibitor and a PI3K Inhibitor is Well Tolerated

This example shows that the combination of an HDAC6 inhibitor and a PI3K inhibitor is well tolerated in mice.

CB-17 SCID mice were treated with Vehicle, GDC-0941 alone (100 mg/kg PO QD), or GDC-0941 (100 mg/kg PO QD) plus **Compound A** (30 mg/kg IP QD). Percent body weight change was determined relative to the start of dosing, and the mean change ±SD was plotted. All treatments were dosed five days per week for 3 cycles. All treatments were well tolerated with no overt evidence of toxicity and complete recovery after minimal body weight loss. See **Figure 5****.**

### Example 10: The Combination of an HDAC6 Inhibitor and a PI3K Inhibitor Reduces Tumor Growth

This example shows that the combination of an HDAC6 inhibitor and a PI3K inhibitor reduces tumor growth in mice.

Mice carrying Granta-519 tumor xenografts were treated with Vehicle, **Compound A** alone (100 mg/kg PO BID), GDC-0941 alone (75 mg/kg PO QD), or GDC-0941 (75 mg/kg PO QD) plus **Compound A** (100 mg/kg PO BID). Tumor volume (mm³) was determined relative to the start of dosing, and the mean volume ±SD was plotted. The combination of **Compound** A and GDC-0941 showed significantly reduced tumor growth relative to either single agent. See **Figure 6****.**

### Example 11: The Combination of an HDAC6 Inhibitor and a PI3K Inhibitor Leads to Further Suppression of Myc Expression

This example shows that the combination of an HDAC6 inhibitor and a PI3K inhibitor leads to further suppression of Myc.

**Figures 7A-B** show images of immunoblots from Mino (**Figure 7A**) and Granta-519 (**Figure 7B**) cells showing that the combination of **Compound A** and either GDC-0941 or CAL-101 led to further suppression of Myc expression, a key transcriptional regulator in cancer, relative to any of the single agents.

## Claims

1. A pharmaceutical combination for use in treating non-hodgkin's lymphoma comprising a therapeutically effective amount of a histone deacetylase 6 (HDAC6) specific inhibitor, wherein the HDAC6 specific inhibitor is: 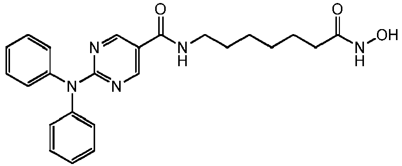 or a pharmaceutically acceptable salt thereof;
and a phosphatidylinositide 3-kinase (PI3K) inhibitor, wherein the PI3K inhibitor is GDC-0941 or GS-1101, or a pharmaceutically acceptable salt thereof.

2. A pharmaceutical combination for use in treating non-hodgkin's lymphoma comprising a therapeutically effective amount of a histone deacetylase 6 (HDAC6) specific inhibitor, wherein the HDAC6 specific inhibitor is: 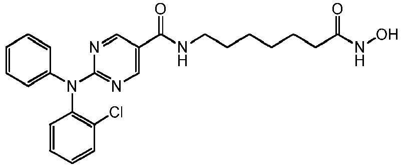 or a pharmaceutically acceptable salt thereof;
and a phosphatidylinositide 3-kinase (PI3K) inhibitor, wherein the PI3K inhibitor is GDC-0941 or GS-1101, or a pharmaceutically acceptable salt thereof.

3. The pharmaceutical combination for the use according to claim 1 or 2, wherein the PI3K inhibitor is GDC-0941 or a pharmaceutically acceptable salt thereof.

4. The pharmaceutical combination for the use according to claim 1 or 2, wherein the PI3K inhibitor is GS-1101 or a pharmaceutically acceptable salt thereof.

5. The pharmaceutical combination for the use according to claim 1 or 2, wherein the combination further comprises a pharmaceutically acceptable carrier.

6. The pharmaceutical combination for the use according to claim 1 or 2, wherein the HDAC6 specific inhibitor is administered at a sub-therapeutic dose.

## Patentansprüche

1. Pharmazeutische Kombination zur Verwendung bei der Behandlung des Non-Hodgkin-Lymphoms, umfassend
eine therapeutisch wirksame Menge eines Histon-Deacetylase 6 (HDAC6)-spezifischen Inhibitors, wobei der HDAC6-spezifische Inhibitor 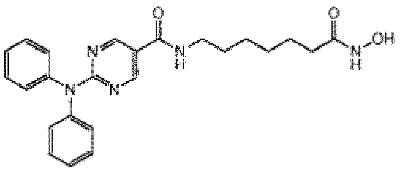 oder ein pharmazeutisch annehmbares Salz davon ist;
und einen Phosphatidylinositid-3-Kinase (P13 K)-Inhibitor, wobei der PI3K-Inhibitor GDC-0941 oder GS-1101 , oder ein pharmazeutisch annehmbares Salz davon ist.

2. Pharmazeutische Kombination zur Verwendung bei der Behandlung des Non-Hodgkin-Lymphoms, umfassend
eine therapeutisch wirksame Menge eines Histon-Deacetylase 6 (HDAC6)-spezifischen Inhibitors, wobei der HDAC6-spezifische Inhibitor 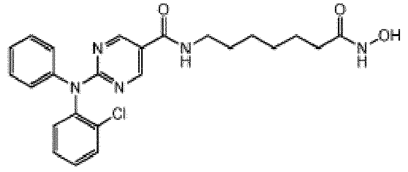 oder ein pharmazeutisch annehmbares Salz davon ist;
und einen Phosphatidylinositid-3-Kinase (PI3K)-Inhibitor, wobei der PI3K-Inhibitor GDC-0941 oder GS-1101 oder ein pharmazeutisch annehmbares Salz davon ist.

3. Pharmazeutische Kombination zur Verwendung nach Anspruch 1 oder 2, bei der der P13K-Inhibitor GDC-0941 oder ein pharmazeutisch verträgliches Salz davon ist.

4. Pharmazeutische Kombination zur Verwendung nach Anspruch 1 oder 2, bei der der P13K-Inhibitor GS-1101 oder ein pharmazeutisch annehmbares Salz davon ist.

5. Pharmazeutische Kombination zur Verwendung nach Anspruch 1 oder 2, wobei die Kombination weiterhin einen pharmazeutisch annehmbaren Träger umfasst.

6. Pharmazeutische Kombination zur Verwendung nach Anspruch 1 oder 2, wobei der HDAC6-spezifische Inhibitor in einer subtherapeutischen Dosis verabreicht wird.

## Revendications

1. Combinaison pharmaceutique destinée à être utilisée dans le traitement du lymphome non hodgkinien comprenant une quantité thérapeutiquement efficace d'un inhibiteur spécifique d'histone désacétylase 6 (HDAC6), dans lequel l'inhibiteur spécifique de HDAC6 est : 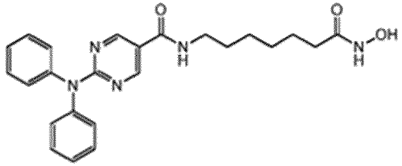 ou un sel pharmaceutiquement acceptable de celui-ci ;
et un inhibiteur de phosphatidylinositide 3-kinase (PI3K), dans laquelle l'inhibiteur de PI3K est GDC-0941 ou GS-1101, ou un sel pharmaceutiquement acceptable de ceux-ci.

2. Combinaison pharmaceutique destinée à être utilisée dans le traitement du lymphome non hodgkinien comprenant une quantité thérapeutiquement efficace d'un inhibiteur spécifique d'histone désacétylase 6 (HDAC6), dans lequel l'inhibiteur spécifique de HDAC6 est : 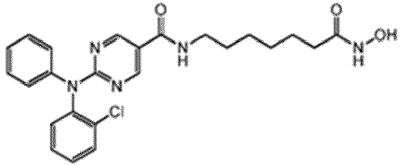 ou un sel pharmaceutiquement acceptable de celui-ci ;
et un inhibiteur de phosphatidylinositide 3-kinase (PI3K), dans laquelle l'inhibiteur de PI3K est GDC-0941 ou GS-1101, ou un sel pharmaceutiquement acceptable de ceux-ci.

3. Combinaison pharmaceutique pour l'utilisation selon la revendication 1 ou 2, dans laquelle l'inhibiteur de PI3K est GDC-0941 ou un sel pharmaceutiquement acceptable de celui-ci.

4. Combinaison pharmaceutique pour l'utilisation selon la revendication 1 ou 2, dans laquelle l'inhibiteur de PI3K est GS-1101 ou un sel pharmaceutiquement acceptable de celui-ci.

5. Combinaison pharmaceutique pour l'utilisation selon la revendication 1 ou 2, dans laquelle la combinaison comprend en outre un excipient pharmaceutiquement acceptable.

6. Combinaison pharmaceutique pour l'utilisation selon la revendication 1 ou 2, dans laquelle l'inhibiteur spécifique de HDAC6 est administré à une dose sous-thérapeutique.
